# EUROPÄISCHE PATENTSCHRIFT

(11) **EP 0 775 143 B1**
(45) Veröffentlichungstag und Bekanntmachung des Hinweises auf die Patenterteilung: **04.09.2002**
(21) Anmeldenummer: 95927636.1
(22) Anmeldetag: 28.07.1995
(51) Int. Cl.: C07D 491/056, A61K 31/55

(54) **3-SUBSTITUIERTE 3H-2,3-BENZODIAZEPINDERIVATE, DEREN HERSTELLUNG UND VERWENDUNG ALS ARZNEIMITTEL**
3-SUBSTITUTED 3H-2,3-BENZODIAZEPINE DERIVATIVES, THEIR PREPARATION AND USE AS MEDICAMENTS
DERIVES SUBSTITUES EN POSITION 3 DE 3H-2,3-BENZODIAZEPINE, LEUR PREPARATION ET LEUR UTILISATION COMME MEDICAMENTS

(30) Priorität: 01.08.1994 DE 4428835
(43) Veröffentlichungstag der Anmeldung: 28.05.1997
(73) Patentinhaber: SCHERING AKTIENGESELLSCHAFT, 13353 Berlin (DE)
(72) Erfinder: HAMORI, Tamás, H-1031 Budapest (HU); TARNAWA, István, H-1147 Budapest (HU); SOLYOM, Sándor, H-1144 Budapest (HU); BERZSENYI, Pál, H-1174 Budapest (HU); BIRKAS FALGLNE, Erzsébet, H-1107 Budapest (HU); ANDRASI, Ferenc, H-1025 Budapest (HU); LING, István, H-1141 Budapest (HU); HASKO, Tibor, H-1043 Budapest (HU); KAPUS, Gábor, H-2120 Dunakeszi (HU); CSUZDL, Emese, H-1046 Budapest (HU); SZOLLOSY, Márta, H-1105 Budapest (HU); ERDO VLDANE, Franciska, H-1039 Budapest (HU); SIMAY, Antal, H-1124 Budapest (HU); ZOLYOMI, Gábor, H-1184 Budapest (HU)
(86) Internationale Anmeldenummer: DE9501029
(87) Internationale Veröffentlichungsnummer: WO96004283

(56) Entgegenhaltungen:
- EP-A- 0 492 485
- FARMACO, EDIZIONE SCIENTIFICA, Bd.40, Nr.12, 1985, PAVIA IT Seiten 942 - 955 F. GATTA ET AL 'Derivatives of 2,3-benzodiazepine'

## Beschreibung

Die Erfindung betrifft 3-substituierte 3H-2,3-Benzodiazepinderivate, deren Herstellung und Verwendung als Arzneimittel.

Es ist bereits aus EP-A-492 485 und Farmaco, Edizione Scientifica, Bd. 40, Nr. 12, 1985 Seiten 942 - 955 bekannt, daß ausgewählte 2,3-Benzodiazepinderivate modulatorische Aktivität an Quisqualat-Rezeptoren besitzen und sich aufgrund dieser Eigenschaft als Arzneimittel zur Behandlung von Krankheiten des zentralen Nervensystems eignen.

Es wurde nun gefunden, daß 3-substituierte 3H-2,3-Benzodiazepinderivate der allgemeinen Formel I 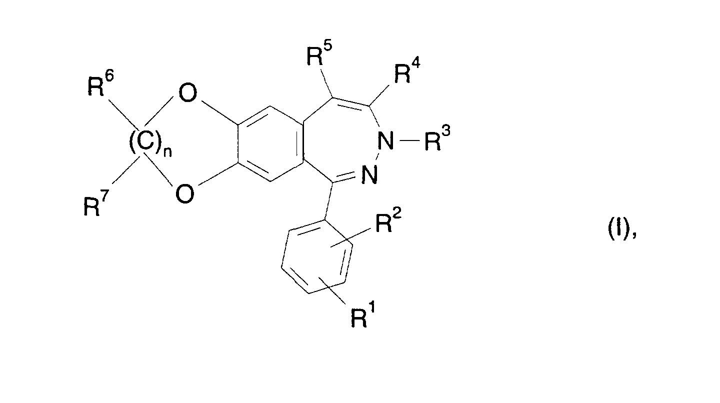 in der
- R¹ und R²: gleich oder verschieden sind und Wasserstoff, C₁ - C₆-Alkyl, Nitro, Halogen, die Gruppe -NR⁸R⁹, -O-C₁₋₄-Alkyloder -CF₃,
- R³: die Gruppe 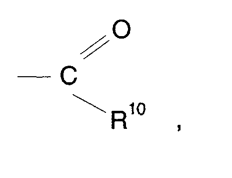
- R⁴: gegebenenfalls mit C₁₋₆-Alkoxy, Halogen oder C₁₋₆-Alkanoyl substituiertes C₁-C₆-Alkyl,
- R⁵: Wasserstoff oder gegebenenfalls mit C₁₋₆-Alkoxy, Halogen oder C₁₋₆-Alkanoyl substituiertes C₁ - C₆-Alkyl,
- R⁶ und R⁷: gleich oder verschieden sind und Wasserstoff, gegebenenfalls mit C₁₋₆-Alkoxy, Halogen oder C₁₋₆-Alkanoyl substituiertes C₁ - C₆-Alkyl oder gegebenenfalls mit C₁₋₆-Alkoxy, Halogen oder C₁₋₆-Alkanoyl substituiertes C₆₋₁₀-Aryl,
- R⁸ und R⁹: gleich oder verschieden sind und Wasserstoff, C₁ - C₆-Alkyl oder die Gruppe 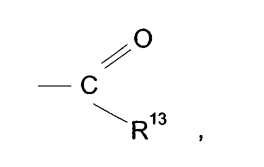
- R¹⁰: Wasserstoff, gegebenenfalls mit C₁₋₆-Alkoxy, Halogen oder C₁₋₆-Alkanoyl substituiertes C₁ - C₆-Alkyl, gegebenenfalls mit C₁₋₆-Alkoxy, Halogen oder C₁₋₆-Alkanoyl substituiertes C₆₋₁₀-Aryl, die Gruppe -NR¹¹R¹², -O-C₁₋₆-Alkyl, C₃₋₇-Cycloalkyl, C₂₋₆-Alkenyl oder -O-C₃₋₇-Cycloalkyl,
- R¹¹ und R¹²: gleich oder verschieden sind und Wasserstoff, gegebenenfalls mit C₁₋₆-Alkoxy, Halogen oder C₁₋₆-Alkanoyl substituiertes C₁ - C₆-Alkyl oder gegebenenfalls mit C₁₋₆-Alkoxy, Halogen oder C₁₋₆-Alkanoyl substituiertes C₆₋₁₀-Aryl,
- R¹³: C₁ - C₆-Alkyl und
- n: für 1, 2 oder 3 steht,
bedeuten sowie deren Isomere und physiologisch verträglichen Salze,
ebenfalls zur Behandlung von Krankheiten des zentralen Nervensystems geeignet sind, wobei die Verbindungen sich gegenüber dem genannten Stand der Technik durch bessere Eigenschaften auszeichnen. Da weder in der 4- noch in der 5-Position ein optisch aktives Zentrum vorhanden ist, sind die Verbindungen der Formel I auch ohne Enantiomerentrennung leicht zugänglich.

Unter Alkyl ist ein geradkettiger oder verzweigter Alkylrest wie beispielsweise Methyl, Ethyl, Propyl, Isopropyl, Butyl, Isobutyl, sek. Butyl, Pentyl, Isopentyl oder Hexyl zu verstehen, der - wie der Aryl-Rest - gegebenenfalls durch C₁ - C₆-Alkoxy, Halogen oder C₁ - C₆-Alkanoyl substituiert sein kann.

Liegt ein halogenierter Alkylrest vor, so kann dieser mehrfach halogeniert bzw. perhalogeniert sein.

Unter Halogen ist Fluor, Chlor, Brom und Jod zu verstehen.

Der Arylrest kann 6-10 Kohlenstoffatome enthalten, wobei Phenyl bevorzugt ist.

Unter Cycloalkyl ist jeweils Cyclopropyl, Cyclobutyl, Cyclopentyl, Cyclohexyl und Cycloheptyl gemeint, insbesondere C₃₋₅-Cycloalkyl.

Die Alkenylreste können geradkettig oder verzweigt sein. Beispielsweise seien genannt: 2-Propenyl, 3-Methyl-2-propenyl, 2-Butenyl, Methallyl, Vinyl.

Alkanoylreste sind Derivate von aliphatischen Carbonsäuren, wie beispielsweise Ameisensäure, Essigsäure, Propionsäure, Buttersäure, Capronsäure, Valeriansäure, Trimethylessigsäure u.a..

Die physiologisch verträglichen Salze leiten sich von anorganischen und organischen Säuren ab. Geeignet sind anorganische Säuren wie beispielsweise Halogenwasserstoffsäuren wie Chlorwasserstoffsäure, Bromwasserstoffsäure, Phosphorsäure, Schwefelsäure oder organische Säuren wie beispielsweise aliphatische oder aromatische Mono- oder Dicarbonsäuren wie Ameisensäure, Essigsäure, Maleinsäure, Fumarsäure, Succinsäure, Milchsäure, Weinsäure, Zitronensäure, Oxalsäure, Glyoxylsäure oder Sulfonsäuren, beispielsweise C₁₋₄-Alkansulfonsäuren wie Methansulfonsäure oder gegebenenfalls durch Halogen oder C₁₋₄-Alkyl substituierte Benzolsulfonsäuren wie p-Toluolsulfonsäure.

Die Verbindungen der Formel I umfassen auch die E- oder Z-Isomeren oder, falls ein chirales Zentrum vorhanden ist, deren Razemate oder Enantiomeren.

Bevorzugte Verbindungen der allgemeinen Formel I sind die, in der R¹ Amino oder Nitro bedeutet.

Besonders bevorzugte Verbindungen der allgemeinen Formel I sind die, in der
- R¹: Nitro oder Amino,
- R²: Wasserstoff.
- R³: die Gruppe 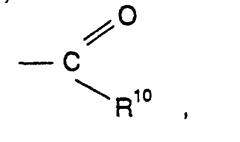
- R⁴: Methyl oder Ethyl,
- R⁵, R⁶ und R⁷: Wasserstoff,
- R¹⁰: Wasserstoff, C₁ - C₆-Alkyl, gegebenenfalls mit Halogen substituiertes Phenyl, die Gruppe -NR¹¹R¹², C₃₋₇-Cycloalkyl, C₂₋₆-Alkenyl, -O-C₁₋₆-Alkyl oder C₁₋₆-Alkyl, das ein- oder mehrfach mit Fluor substituiert ist,
- R¹¹ und R¹²: gleich oder verschieden sind und Wasserstoff, C₁ - C₄-Alkyl oder Phenyl und
- n: 1, 2 oder 3,
bedeuten.

Die Verbindungen der allgemeinen Formel I sowie deren physiologisch verträglichen Salze sind aufgrund ihrer nichtkompetitiven Hemmung der AMPA-Rezeptoren als Arzneimittel verwendbar. Aufgrund ihres Wirkprofils eignen sich die erfindungsgemäßen Verbindungen zur Behandlung von Krankheiten, die durch Hyperaktivität excitatorischer Aminosäuren wie zum Beispiel Glutamat oder Aspartat hervorgerufen werden. Da die neuen Verbindungen als nichtkompetitive Antagonisten excitatorischer Aminosäuren wirken, eignen sie sich insbesondere zur Behandlung von solchen Krankheiten, die über die Rezeptoren excitatorischer Aminosäuren, insbesondere den AMPA-Rezeptor, beeinflußt werden .

Die pharmakologische Wirksamkeit der Verbindungen der Formel I wurde mittels der nachfolgend beschriebenen Teste bestimmt:

Männliche NMRI Mäuse mit einem Gewicht von 18-22 g wurden unter kontrollierten Verhältnissen (6°°-18°° Uhr Hell/Dunkelrythmus, bei freiem Zugang zu Futter und Wasser) gehalten und ihre Zuordnung zu Gruppen wurde randomisiert. Die Gruppen bestanden aus 5 - 16 Tieren. Die Beobachtung der Tiere wurde zwischen 8°° und 13°° Uhr vorgenommen.

AMPA wurde in den linken Ventrikel von frei beweglichen Mäusen gespritzt. Der Applikator bestand aus einer Kanüle mit einer Vorrichtung aus rostfreiem Stahl, die die Tiefe der Injektion auf 3,2 mm begrenzt. Der Applikator war an eine Injektionspumpe angeschlossen. Die Injektionsnadel wurde perpendicular zu der Oberfläche des Schädels nach den Koordinaten von Montemurro und Dukelow eingeführt. Die Tiere wurden bis zum Auftreten von clonischen bzw. tonischen Krämpfen bis zu 180 sec. beobachtet. Die clonischen Bewegungen, die länger als 5 sec. andauern, wurden als Krämpfe gezählt. Der Anfang der clonischen Krämpfe wurde als Endpunkt für die Bestimmung der Krampfschwelle verwendet. Die Dosis, die notwendig war, um die Krampfschwelle um 50% herauf- bzw. herabzusetzen (THRD₅₀) wurde in 4-5 Experimenten bestimmt. Die THRD₅₀ und die Vertrauensgrenze wurde in einer Regressionsanalyse bestimmt.

Die Ergebnisse dieser Versuche zeigen, daß die Verbindung der Formel I und deren Säureadditionssalze funktionelle Störungen des AMPA-Rezeptors beeinflussen. Sie eignen sich daher zu Herstellung von Arzneimitteln zur symptomatischen und präventiven Behandlung von Erkrankungen, die durch Veränderung der Funktion des AMPA-Rezeptor-Komplexes ausgelöst werden.
Die Beharidlung mit den erfindungsgemäßen Verbindungen verhindert bzw. verzögert die infolge der Erkrankung auftretenden Zellschädigungen und funktionellen Störungen und vermindert die dadurch entstehenden Symptome.

Erfindungsgemäß können die Verbindungen verwendet werden zur Behandlung neurologischer und psychiatrischer Störungen, die durch die Überstimulation des AMPA-Rezeptors ausgelöst werden. Zu den neurologischen Erkrankungen , die funktionell und präventiv behandelt werden können, gehören zum Beispiel neurodegenerative Störungen wie Morbus Parkinson, Morbus Alzheimer, Chorea Huntington, Amyotrophe Lateralsklerose und Olivopontocerebelläre Degeneration. Erfindungsgemäss können die Verbindungen zur Prävention des postischämischen Zelluntergangs, des Zelluntergangs nach Hirntrauma, bei Schlaganfall, Hypoxie, Anoxie und Hypoglykämie und zur Behandlung der Senilen Demenz, Aids Demenz, neurologischer Symptome, die mit HIV-Infektionen zusammenhängen, Multiinfarkt Demenz sowie Epilepsie und Muskelspastik verwendet werden. Zu den psychiatrischen Erkrankungen gehören Angstzustände, Schizophrenie, Migräne, Schmerzzustände, sowie die Behandlung von Schlafstörungen und der Entzugssymptomatik nach Drogenmißbrauch wie bei Alkohol-, Kokain-, Benzodiazepinoder Opiat-Entzug. Die Verbindungen können außerdem eine Anwendung in der Prävention der Toleranzentwicklung während der Langzeitbehandlung mit sedativen Arzneimitteln wie zum Beispiel Benzodiazepinen, Barbituraten und Morphin finden. Darüberhinaus können die Verbindungen als Anästhetika (Narkose), Anti-Schmerzmittel oder Antiemetika benutzt werden.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelantine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.
Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5 - 1000 mg, vorzugsweise 50 - 200 mg, wobei die Dosis als einmal zu verabreichende Einzeldosis oder unterteilt in zwei oder mehreren Tagesdosen gegeben werden kann.

Die Herstellung der erfindungsgemäßen Verbindung erfolgt nach an sich bekannten Methoden. Beispielsweise gelangt man zu Verbindungen der Formel I dadurch, daß man eine Verbindung der allgemeinen Formel II 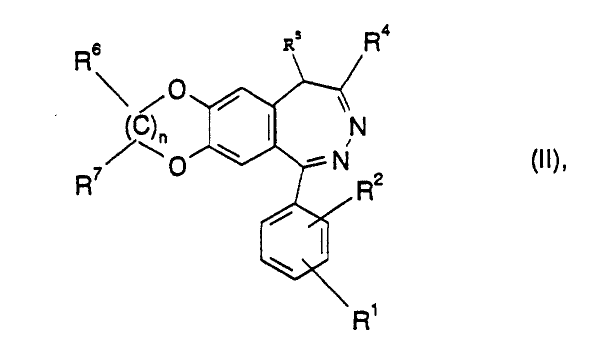 in der
R¹, R², R⁴, R⁵, R⁶, R⁷ und n die in der allgemeinen Formel I angegebenen Bedeutungen haben, acyliert und gegebenenfalls die Nitro-Gruppe an R¹ und/oder R² katalytisch reduziert und anschließend gegebenenfalls acyliert, alkyliert, halogeniert, mit organischen Aminen die Carbamoylgruppe oder mit Alkoholen die Estergruppe einführt, die Isomeren trennt oder die Salze bildet.

Die Acylierung kann mit oder ohne Lösungsmittel bei Raumtemperatur oder erhöhter Temperatur mit den üblichen Acylierungsmitteln durchgeführt werden. Als Acylierungsmittel sind Anhydride oder Säurehalogenide geeignet. Als Anhydride können gemischte oder auch symmetrische Anhydride eingesetzt werden. Wird die Acylierung mit Chlorameisensäureestern wie Chlorameisensäurephenylester vorgenommen, so erhält man durch nachfolgende Reaktion mit primären und sekundären organischen Aminen wie Methylamin die entsprechenden Carbamoylverbindungen bzw. kann durch Umsetzung mit Alkoholen wie Methanol, Ethanol gegebenenfalls in Gegenwart von katalytischen Mengen NaCN die entsprechende Estergruppe eingeführt werden.

Die Reduktion in der Nitrogruppe wird in polaren Lösungsmitteln bei Raumtemperatur oder erhöhter Temperatur durchgeführt. Als Katalysatoren für die Reduktion sind Metalle wie Raney-Nickel oder Edelmetall-katalysatoren wie Palladium oder Platin gegebenenfalls auf Trägern geeignet. Statt Wasserstoff können auch zum Beispiel Ammoniumformiat oder Hydrazin in bekannter Weise benutzt werden. Reduktionsmittel wie Zinn-II-chlorid oder Titan-III-chlorid können ebenso verwendet werden wie komplexe Metallhydride eventuell in Gegenwart von Schwermetallsalzen. Als Reduktionsmittel ist auch Eisen nutzbar. Die Reaktion wird dann in Gegenwart einer Säure wie z.B. Essigsäure oder Ammoniumchlorid gegebenenfalls unter Zusatz eines Lösungsmittels wie zum Beispiel Wasser oder Methanol durchgeführt.

Wird eine Alkylierung einer Aminogruppe gewünscht, so kann nach üblichen Methoden - beispielsweise mit Alkylhalogeniden - oder nach der Mitsonubo Variante durch Umsetzung mit einem Alkohol in Gegenwart von Triphenylphosphin und Azodicarbonsäureester alkyliert werden, oder man unterwirft das Amin einer reduktiven Aminierung mit Aldehyden oder Ketonen gegebenenfalls nacheinander mit zwei verschiedenen Carbonylverbindungen, wobei man gemischte Derivate erhält [Literatur z.B. Verardo et al. Synthesis (1993), 121; Synthesis (1991), 447; Kawaguchi, Synthesis (1985), 701; Micovic et al. Synthesis (1991), 1043].

Die Acylierung einer Aminogruppe erfolgt in üblicher Weise beispielsweise mit einem Säurehalogenid oder Säureanhydrid gegebenenfalls in Gegenwart einer Base wie Dimethylaminopyridin in Lösungsmitteln wie Methylenchlorid, Tetrahydrofuran oder Pyridin, nach der Schotten-Baumann-Variante in wäßriger Lösung bei schwach alkalischem pH-Wert oder durch Umsetzung mit einem Anhydrid in Eisessig.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Kupfer(I)chlorid oder Kupfer(I)bromid in Gegenwart der entsprechenden Säure wie Salzsäure oder Bromwasserstoffsäure oder mit Kaliumjodid umsetzt. Wenn ein organischer Salpetrigsäureester benutzt wird, kann man die Halogene z.B. durch Zusatz von Methylenjodid oder Tetrabrommethan einführen in einem Lösungsmittel wie zum Beispiel Dimethylformamid.
Die Einführung von Fluor gelingt beispielsweise durch Balz Schiemann-Reaktion des Diazoniumtetrafluorborates.
Die Isomerengemische können nach den üblichen Methoden wie beispielsweise Kristallisation, Chromatographie oder Salzbildung in die Enantiomere bzw. E/Z-Isomeren aufgetrennt werden.

Die Herstellung der Salze erfolgt in üblicher Weise, indem man eine Lösung der Verbindung der Formel I mit der äquivalenten Menge oder einem Überschuß einer Säure, die gegebenenfalls in Lösung ist, versetzt und den Niederschlag abtrennt oder in üblicher Weise die Lösung aufarbeitet.

Soweit die Herstellung der Ausgangsverbindungen nicht beschrieben wird, sind diese bekannt oder sie erfolgt analog zu bekannten Verbindungen (z.B. DE 3527117, Brevet d'invention 879404, EP 0492485, HU 191702, FR 2566774, HU 194550, HU 194529, BP 2034706).).

Die nachfolgenden Beispiele erläutern die Herstellung der erfindungsgemäßen Verbindungen.

### Beispiel 1

### 7-Acetyl-5-(4-nitrophenyl)-8-methyl-7H-1,3-dioxolo[4,5-h][2,3]-benzodiazepin

Eine Suspension von 1,0 g (3,1 mmol) 5-(4-Nitrophenyl)-8-methyl-9H-7H-1,3-dioxolo [4,5-h][2,3]-benzodiazepin werden in 10 ml Acetanhydrid 7 Stunden auf 130- 140 °C erwärmt. Die Lösung wird auf Eis gegeben, der Niederschlag wird abgesaugt. Man erhält 0,99 g (87 % der Theorie) eines gelben Pulvers. Eine Flash Chromatographie dieses Rohprodukts über Kieselgel 60 mit Benzol/Essigester (4 : 1) als Laufmittel ergibt nach Zusammenfassen der entsprechenden Fraktionen und Einengen 0,78 g (70 % der Theorie) 7-Acetyl-5-(4-nitrophenyl)-8-methyl-7H-1,3-dioxolo [4,5-h][2,3]-benzodiazepin als Feststoff mit einem Schmelzpunkt von 200 - 202 °C. Eine Probe wird aus Ethanol umkristallisiert und hat dann einen Schmelzpunkt von 205 - 207 °C (leichte Zersetzung).

In analoger Verfahrensweise werden hergestellt:
7-Acetyl-5-(4-N-acetylaminophenyl)-8-methyl-7H-1,3-dioxolo [4,5-h] [2,3]-benzodiazepin

### Beispiel 2

### 7-Acetyl-5-(4-aminophenyl)-8-methyl-7H-1,3-dioxolo [4,5-h][2,3]-benzodiazepin

Zu einer Suspension von 0,6 g (1,64 mmol) von 7-Acetyl-5-(4-nitrophenyl)-8-methyl-7H-1,3-dioxolo [4,5-h][2,3]-benzodiazepin in 40 ml Methanol werden 0,1 g Raney Nickel und 0,25 ml (4,93 mmol) 98 %iges Hydrazinhydrat gegeben. Die Mischung wird 0,5 Stunden gerührt. Das Ausgangsmaterial löst sich innerhalb von wenigen Minuten auf. Nachdem die Reaktion beendet ist, wird vom Katalysator abfiltriert. Das Filtrat wird am Vakuum eingeengt. Der Rückstand wird in Wasser ausgerührt, abgesaugt und mit Wasser nachgewaschen. Man erhält 0,49 g 7-Acetyl-5-(4-aminophenyl)-8-methyl-7H-1,3-dioxolo [4,5-h][2,3]-benzo- diazepin mit einem Schmelzpunkt von 175 - 180 °C. Umkristallisation dieses Rohprodukts aus 50 %-igem wäßrigen Ethanol ergibt 0,46 g (79 %) 7-Acetyl-5-(4-amino-phenyl)-8-methyl-7H-1,3-dioxolo [4,5-h][2,3]-benzodiazepin mit einem Schmelzpunkt von 208 - 210 °C.
¹H-NMR (CDCl₃): d: 2,23 (s, 3H), 2,26 (br s, 3H), 4,02 (br s, 2H), 5,97 (br s, 1H), 6,05 (br s, 1H), 6,32 (br s, 1H), 6,64 (d, 2H), 6,74 (s, 2H), 7,33 (d, 2H):
Elementaranalyse: Berechnet für C₁₉H₁₇N₃O₃·H₂O (353,36): C = 64,68 %; H = 5,42 %; N = 11,89 %; Gef: C = 64,74 %; H = 4,89 %; N = 11,92 %;

### Beispiele 3 - 18

Analog Beispiel 1 erhält man die in der Tabelle 1 beschriebenen Verbindungen der Formel I, worin R⁵, R⁶ und R⁷ Wasserstoff, n = 1 und R⁴ = Methyl bedeutet

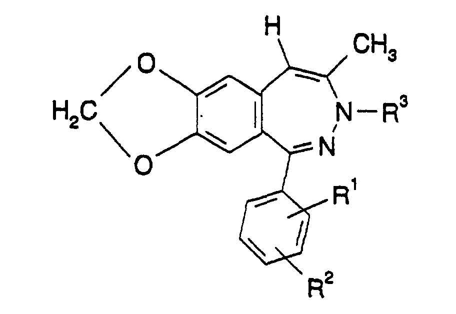

**Tabelle 1**

| **Beispiel Nr.** | **R**^{**1**} | **R**^{**2**} | **R**^{**3**} | **M.p., °C** |
|---|---|---|---|---|
| **3** | 4-NO₂ | H | Propionyl | 206-208 |
| **4** | 3-NO₂ | H | Acetyl | 198-200 |
| **5** | 2-NO₂ | H | Acetyl | 192-194 |
| **6** | 4-NO₂ | H | Pivaloyl | 225-227 |
| **7** | 4-NO₂ | H | Trifluoracetyl | 213-215 |
| **8** | 2-Cl | H | Acetyl | 197-198 |
| **9** | 2-CH₃ | H | Acetyl | 182-184 |
| **10** | 4-OCH₃ | H | Acetyl | 173-176 |
| **11** | 3-OCH₃ | H | Acetyl | 120-122 |
| **12** | 3-Cl | H | Acetyl | 130-132 |
| **13** | 4-F | H | Acetyl | 208-210 |
| **14** | 4-NO₂ | 2-Br | Acetyl | Öl |
| **15** | 4-OCH₃ | 2-iPropyl | Acetyl | 96-98 |
| **16** | 4-OCH₃ | 3-F | Acetyl | 196-198 |
| **17** | 3-CF₃ | H | Acetyl | 210-212 |
| **18** | 4-CF₃ | H | Acetyl | 122-124 |

### Beispiel 19

### 7-Cyclopropancarbonyl-8-methyl-5-phenyl-7H-1,3-dioxolo [4,5-h] [2,3]-benzodiazepin

Eine Suspension von 0,56 g (2,0 mmol) 8-Methyl-5-phenyl-9-H-1,3-dioxolo [4,5-h] [2,3] benzodiazepin und 0,41 g (3,0 mmol) Kaliumcarbonat in 15 ml Benzol werden zum Sieden erhitzt, und es werden 0,31 g (3,0 mmol) Cyclopropancarbonylchlorid hinzugefügt. Bei dieser Temperatur wird 1,5 Stunden nachgerührt und anschließend filtriert. Nach Abzug des Lösungsmittels wird der Rückstand an Kieselgel 60 mit Hexan/Ethylacetat 2:1 als Elutionsmittel chromatographiert. Nach Kristallisation der Hauptfraktion aus Ethanol erhält man 0,35 g (50%) der Titelverbindung mit dem Schmelzpunkt 165-166 °C.

### Beispiel 20-26

Analog Beispiel 19 erhält man die in Tabelle 2 beschriebenen Verbindungen der Formel 1, worin R⁵, R⁶ und R⁷ Wasserstoff, n = 1 und R⁴ = Methyl bedeuten

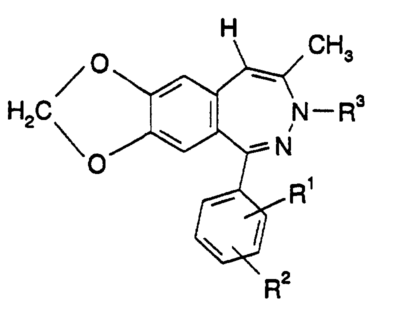

**Tabelle 2**

| **Beispiel Nr.** | **R**^{**1**} | **R**^{**2**} | **R**^{**3**} | **M.p., °C** |
|---|---|---|---|---|
| **20** | 4-NO₂ | H | Benzoyl | >212°C |
| **21** | 4-NO₂ | H | 3-Chlorbenzoyl | 260-263°C |
| **22** | 4-NO₂ | H | Cyclopropancarbonyl | weiterverarbeitet |
| **23** | 4-NO₂ | H | Methacryloyl | weiterverarbeitet |
| **24** | 4-NO₂ | H | Isobutyryl | weiterverarbeitet |
| **25** | 4-NO₂ | H | Isovaleryl | weiterverarbeitet |
| **26** | 4-NO₂ | H | Butyryl | weiterverarbeitet |

### Beispiel 27

### 7-Formyl-5-(4-nitrophenyl)-8-methyl-7H-1,3-dioxolo [4,5-h] [2,3] benzodiazepin

Analog Beispiel 1 erhält man die Titelverbindung mit einer Mischung aus Acetanhydrid und 98% Ameisensäure 3:1. Nach Chromatographie an Kieselgel mit Benzol/Ethylacetat 4:1 als Elutionsmittel erhält man braune Kristalle, Schmelzpunkt 123-127 °C (36%).

### Beispiel 28

### 7-Ethoxycarbonyl-5-(4-nitrophenyl)-8-methyl-7H-1,3-dioxolo [4,5] [2,3] benzodiazepin

5-(4-Nitrophenyl)-8-methyl-9H-1,3-dioxolo [4,5-h] [2,3]-Benzodiazepin (0,323 g / 1,0 mmol), wasserfreies Kaliumcarbonat (0,28 g, 2,0 mmol), Chlorameisensäureethylester (0,12 ml, 1,2 mmol) und wasserfreies Benzol werden unter Rühren am Rückfluß erhitzt. Jeweils nach 1 Stunde und nach 12 Stunden werden Chlorameisensäureethylester (0,12 ml) und Kaliumcarbonat (0,28 g) hinzugefügt. Nach 18 Stunden wird abfiltriert, konzentriert und der Rückstand über Kieselgel mit Benzol:Ethylacetat als Elutionsmittel chromatographiert. Man erhält 92 mg gelbe amorphe Kristalle (23%), die ohne Reinigung weiterverarbeitet werden.

### Beispiel 29

### 8-Methyl-7-methylcarbamoyl-5-(4-nitrophenyl)-7H-1,3-dioxolo [4,5-h] [2,3] benzodiazepin

**A:** Eine Mischung von 5-(4-Nitrophenyl)-8-methyl-9H-1,3-dioxolo [4,5-h] [2,3] benzodiazepin (1,61 g, 5,0 mmol), Chlorameisensäurephenylester (1,5ml, 12,0 mmol), Kaliumcarbonat (1,4 g, 10 mmol) und Benzol (50 ml) werden unter Rühren 1,5 Stunden am Rückfluß erhitzt. Die Reaktionsmischung wird heiß filtriert, eingeengt und der Rückstand mit 15 ml Essigester ausgerührt. Das Filtrat wird eingeengt und ohne weitere Reinigung in den nächsten Reaktionsschritt eingesetzt.

**B**: Der nach A erhaltene Rückstand wird mit 10 ml DMF und 3 ml einer 40%iger Lösung von Methylamin in Wasser versetzt, auf 100 °C erhitzt und nach 16 Stunden in Wasser gegeben. Nach Extraktion mit Chloroform wird in üblicher Weise aufgearbeitet und man erhält 0,307 g der Titelverbindung (16%).

### Beispiel 30

### 8-Methyl-7-methoxycarbonyl-5-(4-nitrophenyl)-7H-1,3-dioxolo [4,5-h] [2,3] benzodiazepin

1,85 g (3,9 mmol) des nach Beispiel 29, Reaktionsschritt A erhaltenen Produktes werden mit 90 ml wasserfreiem Methanol und 15 ml einer 50%igen Lösung von Methylamin in Methanol gemischt. Es wird eine katalytische Menge NaCN hinzugefügt und 18 Stunden auf 90 °C erhitzt. Es wird in üblicher Weise aufgearbeitet. Nach Chromatographie mit Benzol/Ethylacetat 2:1 erhält man 0,44 g (30%) der Titelverbindung.

### Beispiel 31-47

Analog Beispiel 2 erhält man die in der Tabelle 3 beschriebenen Verbindungen der Formel I, worin R⁴ = Methyl, n = 1, R⁵, R⁶ und R⁷ Wasserstoff bedeuten

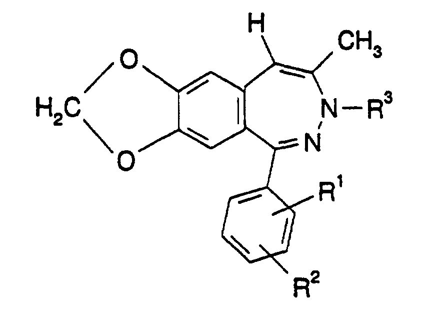

**Tabelle 3**

| **Beispiel Nr.** | **R**^{**1**} | **R**^{**2**} | **R**^{**3**} | **M.p., °C** |
|---|---|---|---|---|
| **31** | 4-NH₂ | H | Formyl | mit 1/2 eq. H₂O, 141-145 ohne Wasser 186 |
| **32** | 4-NH₂ | H | Propionyl | ohne Wasser:186, 170-172 |
| **33** | 3-NH₂ | H | Acetyl | 196-198 |
| **34** | 4-NH₂ | H | Benzoyl | 206-207 |
| **35** | 4-NH₂ | H | 3-Chlorbenzoyl | 132-134 |
| **36** | 4-NH₂ | H | Cyclopropancarbonyl | 215-217 |
| **37** | 4-NH₂ | H | Methacryloyl | 127-132 |
| **38** | 4-NH₂ | H | Isobutyryl | 130-135 |
| **39** | 4-NH₂ | H | Isovaleryl | 100-103 |
| **40** | 4-NH₂ | H | Butyryl | 114-116 |
| **41** | 4-NH₂ | H | Pivaloyl | 206-208 |
| **42** | 4-NH₂ | H | Trifluoracetyl | 142-145 |
| **43** | 2-NH₂ | H | Acetyl | 157-158 |
| **44** | 4-NH₂ | 2-Br | Acetyl | 250-252 |
| **45** | 4-NH₂ | H | Methylcarbamoyl | 157-167 |
| **46** | 4-NH₂ | H | Ethoxycarbonyl | 142-144 |
| **47** | 4-NH₂ | H | Methoxycarbonyl | 144-148 |

### Beispiel 48

### 7-Acetyl-5-(4-nitrophenyl)-8-ethyl-7H-1,3-dioxolo [4,5-h] [2,3] benzodiazepin

**A**: 7-Ethyl-5-(4-nitrophenyl)-1,3-dioxolo [4,5g] isochroman
   Man reduziert 1-(3-Benzdioxol-5-yl)-butan-2-on (Nichols et al. J. Med. Chem. 1986, 29, 2009) zum entsprechenden Isobutanol und setzt dieses mit 4-Nitrobenzaldehyd nach dem Ungarischen Patent HU 194550 (C.A. 105, 1986, 226357V) zur Titelverbindung um. Ausbeute 85 %, Schmelzpunkt 116-118 °C (Methanol).
**B:** 7-Ethyl-5-(4-nitrophenyl)-1,3-dioxolo [4,5-g] [2] benzopyryliumperchlorat
   Die nach Reaktionsschritt A erhaltene Isochroman-Verbindung wird oxidiert nach dem Ungarischen Patent HU 194529 (C.A. 105, 1986, 152712h) zu dem entsprechenden Diketon, das mit 70%iger HClO₄ in das Benzopyryliumsalz überführt wird. Ausbeute 50%, Schmelzpunkt 243-245°C (Zers.)
**C:** 8-Ethyl-5-(4-nitrophenyl)-9H-1,3-dioxolo [4,5-h] [2,3] benzodiazepin
   Das nach B erhaltene Pyryliumsalz reagiert mit Hydrazinhydrat nach dem Britischen Patent BP 20 34 706 (C.A. 94, 1981, 103443S) zum 9-H-2,3-Benzodiazepin. Ausbeute 88 %, Schmelzpunkt 218-220 °C (DMF).
**D**: Das nach Reaktionsschritt C erhaltene Produkt wird analog Beispiel 1 acetyliert. Man erhält die Titelverbindung mit dem Schmelzpunkt 132-134 °C (Ethanol), Ausbeute 33 %.

### Beispiel 49

### 7-Acetyl-5-(4-aminophenyl)-8-ethyl-7H-1,3-dioxolo [4,5-h] [2,3] benzodiazepin

Die nach Beispiel 48 erhaltene Verbindung wird analog Beispiel 2 reduziert. Ausbeute 45%, Schmelzpunkt 136-138°C (Ethanol/Wasser 1:1).

### Beispiel 50

### 8-Acetyl-9-methyl-6-(4-nitrophenyl)-8H-2,3-dihydro-1,4-dioxano [2,3-h] [2,3] benzodiazepin

**A:** 6,7-Dihydroxy-3-methyl-1-(4-nitrophenyl)-2-benzopyrylium-perchlorat
   44,0 g 3-Methyl-6,7-dimethoxy-1-(4-nitrophenyl)-2-benzopyrylium-perchlorat (C.A. 105, 1986, 152712h) werden zu einer Lösung von 46,3 g AlCl₃ in 170 ml Nitromethan gegeben und 4 Stunden zum Sieden erhitzt. Das Lösungsmittel wird entfernt, der Rückstand mit 500 ml gekühlter 50%iger HCl-Lösung behandelt und das erhaltene Produkt mit kaltem Wasser gewaschen. Man erhält 43,5 g Rohprodukt, das mit 130 ml Essigsäure versetzt, zum Sieden erhitzt und mit 13,3 ml 70%iger HClO₄ versetzt wird. Nach Abkühlen erhält man 30,0 g der Titelverbindung (73%) mit dem Schmelzpunkt 253-255 °C (Zers. Essigester).
**B:** 7,8-Dihydroxy-4-methyl-1-(4-nitrophenyl)-5H-2,3-benzodiazepin
   Die Verbindung des Reaktionsschrittes A wird mit Hydrazinhydrat analog C.A. 94, 1981, 1034435 zur Titelverbindung umgesetzt, Schmelzpunkt 254-256 °C (Zersetzung).
**C:** 9-Methyl-6-(4-nitrophenyl)-10H-2,3-dihydro-1,4-dioxano [2,3-h] [2,3] benzodiazepin
   Zu einer Suspension von 3,0 g der nach Reaktionsschritt B erhaltenen Verbindung in 37 ml trockenem DMF werden 4,8 g KF und 1,12 ml 1,2-Dibromethan gegeben und die Mischung 1 Stunde bei 110-120 °C gerührt. Nach dem Abkühlen wird die Reaktionsmischung in 250 ml Wasser gegeben, das Produkt abfiltriert, mit Wasser gewaschen und in üblicher Weise aufgearbeitet. Man erhält 1,42 g (44%) der Titelverbindung mit dem Schmelzpunkt 228-230 °C (Zers.).
**D**: Die nach C erhaltene Verbindung wird analog Beispiel 1 acetyliert, Ausbeute 82%, Schmelzpunkt 226-228 °C (Zers.).

### Beispiel 51

### 8-Acetyl-9-methyl-6-(4-aminophenyl)-8H-2,3-dihydro-1,4-dioxano [2,3-h] [2,3] benzodiazepin

Die nach Beispiel 50 erhaltene Verbindung wird analog Beispiel 2 reduziert. Ausbeute 72%, Schmelzpunkt 231-233 °C (Zers.).

### Beispiel 52

### 9-Acetyl-10-methyl-7-(4-nitrophenyl)-2,3,4,9-tetrahydro-1,5-dioxepino-[2,3-h][2,3] benzodiazepin

**A**: 10-Methyl-7-(4-nitrophenyl)-2,3,4,11-tetrahydro-1,5-dioxepino [2,3-h] [2,3] benzodiazepin
   Das im Reaktionsschritt C des Beispiels 50 beschriebene Verfahren wird mit 1,3-Dibrompropan durchgeführt und man erhält die Titelverbindung in 40%iger Ausbeute mit dem Schmelzpunkt 204-206 °C (DMF/Wasser 10:1).
**B**: Die Acetylierung erfolgt analog Beispiel 1 und man erhält die Titelverbindung in 65%iger Ausbeute mit dem Schmelzpunkt von 202-204°C.

### Beispiel 53

### 9-Acetyl-10-methyl-7-(4-aminophenyl)-2,3,4,9-tetrahydro-1,5-dioxepino [2,3-h] [2,3] benzodiazepin

Die nach Beispiel 52 erhaltene Verbindung wird analog Beispiel 2 reduziert und man erhält die Titelverbindung in 66%iger Ausbeute mit dem Schmelzpunkt von 183 - 184°C.

## Patentansprüche

1. Verbindungen der Formel I, 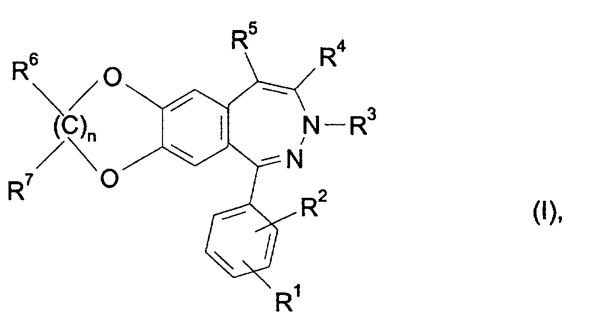 worin
R¹ und R² gleich oder verschieden sind und Wasserstoff, C₁ - C₆-Alkyl, Nitro, Halogen, die Gruppe -NR⁸R⁹, oder O-C₁₋₄-Alkyl, oder -CF₃
R³ die Gruppe 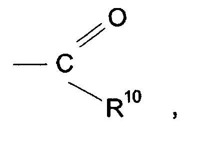
R⁴ gegebenenfalls mit C₁₋₆-Alkoxy, Halogen oder C₁₋₆-Alkanoyl substituiertes C₁-C₆-Alkyl,
R⁵ Wasserstoff oder gegebenenfalls mit C₁₋₆-Alkoxy, Halogen oder C₁₋₆-Alkanoyl substituiertes C₁ - C₆-Alkyl,
R⁶ und R⁷ gleich oder verschieden sind und Wasserstoff, gegebenenfalls mit C₁₋₆-Alkoxy, Halogen oder C₁₋₆-Alkanoyl substituiertes C₁ - C₆-Alkyl oder gegebenenfalls mit C₁₋₆-Alkoxy, Halogen oder C₁₋₆-Alkanoyl substituiertes C₆₋₁₀-Aryl,
R⁸ und R⁹ gleich oder verschieden sind und Wasserstoff, C₁ - C₆-Alkyl oder die Gruppe 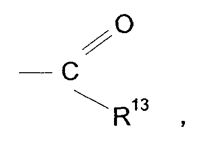
R¹⁰ Wasserstoff, gegebenenfalls mit C₁₋₆-Alkoxy, Halogen oder C₁₋₆-Alkanoyl substituiertes C₁ - C₆-Alkyl, gegebenenfalls mit C₁₋₆-Alkoxy, Halogen oder C₁₋₆-Alkanoyl substituiertes C₆₋₁₀-Aryl, die Gruppe -NR¹¹R¹², -O-C₁₋₆-Alkyl, C₃₋₇-Cycloalkyl, C₂₋₆-Alkenyl oder -O-C₃₋₇-Cycloalkyl,
R¹¹ und R¹² gleich oder verschieden sind und Wasserstoff, gegebenenfalls mit C₁₋₆-Alkoxy, Halogen oder C₁₋₆-Alkanoyl substituiertes C₁ - C₆-Alkyl oder gegebenenfalls mit C₁₋₆-Alkoxy, Halogen oder C₁₋₆-Alkanoyl substituiertes C₆₋₁₀-Aryl,
R¹³ C₁ - C₆-Alkyl und
n für 1, 2 oder 3 steht,
bedeuten sowie deren physiologisch verträglichen Salze und Isomere.

2. Verbindungen der Formel I, gemäß Anspruch 1, worin
R¹ Nitro oder Amino,
R² Wasserstoff, C₁ - C₆-Alkyl, Nitro, Halogen, die Gruppe -NR⁸R⁹, -O-C₁₋₄-Alkyl oder -CF₃,
R³ die Gruppe 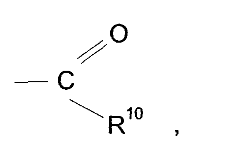
R⁴ gegebenenfalls mit C₁₋₆-Alkoxy, Halogen oder C₁₋₆-Alkanoyl substituiertes C₁-C₆-Alkyl,
R⁵ Wasserstoff oder gegebenenfalls mit C₁₋₆-Alkoxy, Halogen oder C₁₋₆-Alkanoyl substituiertes C₁ - C₆-Alkyl,
R⁶ und R⁷ gleich oder verschieden sind und Wasserstoff, gegebenenfalls mit C₁₋₆-Alkoxy, Halogen oder C₁₋₆-Alkanoyl substituiertes C₁ - C₆-Alkyl oder gegebenenfalls mit C₁₋₆-Alkoxy, Halogen oder C₁₋₆-Alkanoyl substituiertes C₆₋₁₀-Aryl,
R⁸ und R⁹ gleich oder verschieden sind und Wasserstoff, C₁ - C₆-Alkyl oder die Gruppe 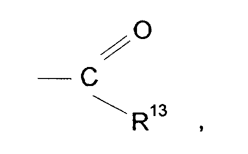
R¹⁰ Wasserstoff, gegebenenfalls mit C₁₋₆-Alkoxy, Halogen oder C₁₋₆-Alkanoyl substituiertes C₁ - C₆-Alkyl, gegebenenfalls mit C₁₋₆-Alkoxy, Halogen oder C₁₋₆-Alkanoyl substituiertes C₆₋₁₀-Aryl, die Gruppe -NR¹¹R¹², -O-C₁₋₆-Alkyl, C₃₋₇-Cycloalkyl, C₂₋₆-Alkenyl oder -O-C₃₋₇-Cycloalkyl,
R¹¹ und R¹² gleich oder verschieden sind und Wasserstoff, gegebenenfalls mit C₁₋₆-Alkoxy, Halogen oder C₁₋₆-Alkanoyl substituiertes C₁ - C₆-Alkyl oder gegebenenfalls mit C₁₋₆-Alkoxy, Halogen oder C₁₋₆-Alkanoyl substituiertes C₆₋₁₀-Aryl,
R¹³ C₁ - C₆-Alkyl und
n für 1, 2 oder 3 steht,
bedeuten, sowie deren physiologisch verträglichen Salze und Isomere.

3. 7-Acetyl-5-(4-aminophenyl)-8-methyl-7H-1,3-dioxolo[4,5-h][2,3]benzodiazepin
7-Methylcarbamoyl-5-(4-aminophenyl)-8-methyl-7H-1,3-dioxolo[4,5-h][2,3]benzodiazepin, gemäß Anspruch 1.

4. Verbindungen der allgemeinen Formel I, gemäß Anspruch 1, worin
R¹ Nitro oder Amino,
R² Wasserstoff.
R³ die Gruppe 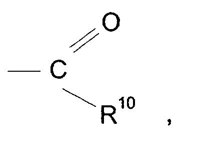
R⁴ x Methyl oder Ethyl,
R⁵, R⁶ und R⁷ Wasserstoff,
R¹⁰ Wasserstoff, C₁ - C₆-Alkyl, gegebenenfalls mit Halogen substituiertes Phenyl, die Gruppe -NR¹¹R¹², C₃₋₇-Cycloalkyl, C₂₋₆-Alkenyl, -O-C₁₋₆-Alkyl oder C₁₋₆-Alkyl, das ein- oder mehrfach mit Fluor substituiert ist,
R¹¹ und R¹² gleich oder verschieden sind und Wasserstoff, C₁ - C₄-Alkyl oder Phenyl und
n 1, 2 oder 3 ist, bedeuten, sowie deren physiologisch verträglichen Salze und Isomere.

5. Verfahren zur Herstellung von Verbindungen der allgemeinen Formel I, gemäß Anspruch 1, **dadurch gekennzeichnet, daß** man eine Verbindung der allgemeinen Formel II 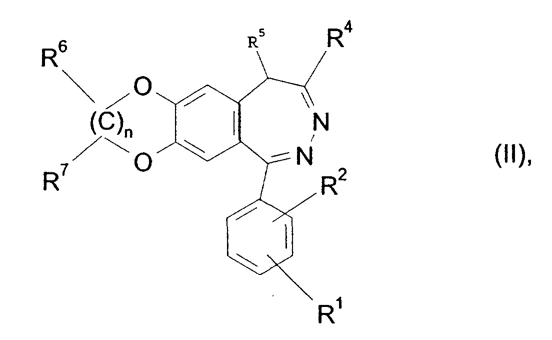 in der
R¹, R², R⁴, R⁵, R⁶, R⁷ und n die in der allgemeinen Formel I angegebenen Bedeutungen haben, acyliert und gegebenenfalls die Nitro-Gruppe an R¹ und/oder R² katalytisch reduziert und anschließend gegebenenfalls acyliert, alkyliert, halogeniert, mit organischen Aminen die Carbamoylgruppe oder mit Alkoholen die Estergruppe einführt, die Isomeren trennt oder die Salze bildet.

6. Arzneimittel enthaltend eine Verbindung der allgemeinen Formel I gemäß einem der Ansprüche 1 bis 4 und einen oder mehrere pharmazeutisch übliche Träger- und/oder Hilfsstoffe.

7. Verwendung einer Verbindung der allgemeinen Formel I gemäß den Ansprüchen 1 bis 4 zur Herstellung eines Arzneimittels zur Behandlung einer Erkrankung, die durch Hyperaktivität excitatorischer Aminosäuren beeinflußt wird.

## Claims

1. Compounds of formula I, 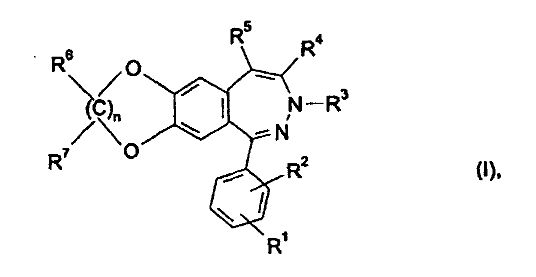 in which
R¹ and R² are the same or different and mean hydrogen, C₁-C₆ alkyl, nitro, halogen, the group -NR⁸R⁹, O-C₁₋₄ alkyl or -CF₃,
R³ means the group 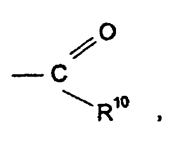 R⁴ means unsubstituted or C₁₋₆ alkoxy-, halogen- or C₁₋₆ alkanoyl-substituted C₁-C₆ alkyl,
R⁵ means hydrogen or unsubstituted or C₁₋₆ alkoxy-, halogen- or C₁₋₆ alkanoyl-substituted C₁-C₆ alkyl,
R⁶ and R⁷ are the same or different and mean hydrogen, unsubstituted or C₁₋₆ alkoxy-, halogen- or C₁₋₆ alkanoyl-substituted C₁-C₆ alkyl or unsubstituted or C₁₋₆ alkoxy-, halogen- or C₁₋₆ alkanoyl-substituted C₆₋₁₀ aryl,
R⁸ and R⁹ are the same or different and mean hydrogen, C₁-C₆ alkyl or the group 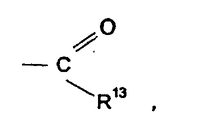 R¹⁰ means hydrogen, unsubstituted or C₁₋₆ alkoxy-, halogen- or C₁₋₆ alkanoyl-substituted C₁-C₆ alkyl, unsubstituted or C₁₋₆ alkoxy-, halogen- or C₁₋₆ alkanoyl-substituted C₆₋₁₀ aryl, the group -NR¹¹R¹², -O-C₁₋₆ alkyl, C₃₋₇ cycloalkyl, C₂₋₆ alkenyl or -O-C₃₋₇ cycloalkyl,
R¹¹ and R¹² are the same or different and mean hydrogen, unsubstituted or C₁₋₆ alkoxy-, halogen- or C₁₋₆ alkanoyl-substituted C₁-C₆ alkyl or unsubstituted or C₁₋₆ alkoxy-, halogen- or C₁₋₆ alkanoyl-substituted C₆₋₁₀ aryl,
R¹³ means C₁-C₆ alkyl and
n stands for 1, 2 or 3,
as well as their physiologically compatible salts and isomers.

2. Compounds of formula 1, according to Claim 1, in which
R¹ means nitro or amino,
R² means hydrogen, C₁-C₆ alkyl, nitro, halogen, the group -NR⁸ R⁹, -O-C₁₋₄ alkyl or -CF₃,
R³ means the group 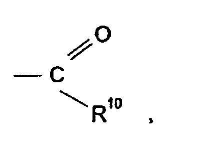 R⁴ means unsubstituted or C₁₋₆ alkoxy-, halogen- or C₁₋₆ alkanoyl-substituted C₁-C₆ alkyl,
R⁵ means hydrogen or unsubstituted or C₁₋₆ alkoxy-, halogen- or C₁₋₆ alkanoyl-substituted C₁-C₆ alkyl,
R⁶ and R⁷ are the same or different and mean hydrogen, unsubstituted or C₁₋₆ alkoxy-, halogen- or C₁₋₆ alkanoyl-substituted C₁-C₆ alkyl or unsubstituted or C₁₋₆ alkoxy-, halogen- or C₁₋₆ alkanoyl-substituted C₆₋₁₀ aryl,
R⁸ and R⁹ are the same or different and mean hydrogen, C₁-C₆ alkyl or the group 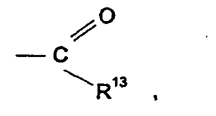 R¹⁰ means hydrogen, unsubstituted or C₁₋₆ alkoxy-, halogen- or C₁₋₆ alkanoyl-substituted C₁-C₆ alkyl, unsubstituted or C₁₋₆ alkoxy-, halogen- or C₁₋₆ alkanoyl-substituted C₆₋₁₀ aryl, the group -NR¹¹R¹², -O-C₁₋₆ alkyl, C₃₋₇ cycloalkyl, C₂₋₆ alkenyl or -O-C₃₋₇ cycloalkyl,
R¹¹ and R¹² are the same or different and mean hydrogen, unsubstituted or C₁₋₆ alkoxy-, halogen- or C₁₋₆ alkanoyl-substituted C₁-C₆ alkyl or unsubstituted or C₁₋₆ alkoxy-, halogen- or C₁₋₆ alkanoyl-substituted C₆₋₁₀ aryl,
R¹³ means C₁-C₆ alkyl and
n stands for 1, 2 or 3,
as well as their physiologically compatible salts and isomers.

3. 7-Acetyl-5-(4-aminophenyl)-8-methyl-7H-1,3dioxolo [4,5-h][2,3] benzodiazepine
7-methylcarbamoyl-5-(4-aminophenyl)-8-methyl-7H-1,3-dioxolo[4,5-h][2,3] benzodiazepine, according to Claim 1.

4. Compounds of general formula 1, according to Claim 1, in which
R¹ means nitro or amino,
R² means hydrogen,
R³ means the group 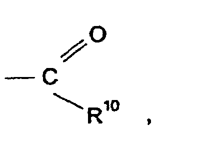 R⁴ means methyl or ethyl,
R⁵, R⁶ and R⁷ mean hydrogen,
R¹⁰ means hydrogen, C₁-C₆ alkyl, phenyl optionally substituted with halogen, the group -NR¹¹R¹², C₃₋₇ cycloalkyl, C₂₋₆ alkenyl, -O-C₁₋₆ alkyl or C₁₋₆ alkyl which is substituted one or more times with fluorine,
R¹¹ and R¹² are the same or different and mean hydrogen, C₁-C₄ alkyl or phenyl, and
n is 1, 2 or 3, as well as their physiologically compatible salts and isomers.

5. Process for the production of compounds of general formula I, according to Claim 1, **characterized in that** a compound of general formula II 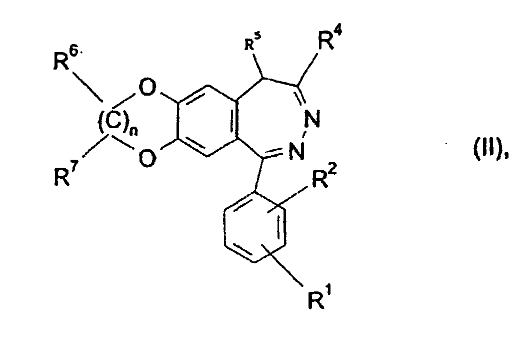 in which R¹, R², R⁴, R⁵, R⁶, R⁷ and n have the meanings indicated in general formula I, is acylated, and optionally the nitro group at R¹ and/or R² is catalytically reduced and then the compound is optionally acylated, alkylated, or halogenated, the carbamoyl group is introduced using organic amines or the ester group using alcohols, the isomers are separated or the salts are formed.

6. Phamaceutical agent that contains a compound of general formula I according to any of Claims 1 to 4 and one or more pharmaceutically customary carriers and/or excipients.

7. The use of a compound of general formula I according to Claims 1 to 4 for the production of a pharmaceutical agent for the treatment of a disorder which is influenced by hyperactivity of excitatory amino acids.

## Revendications

1. Composés de formule I, 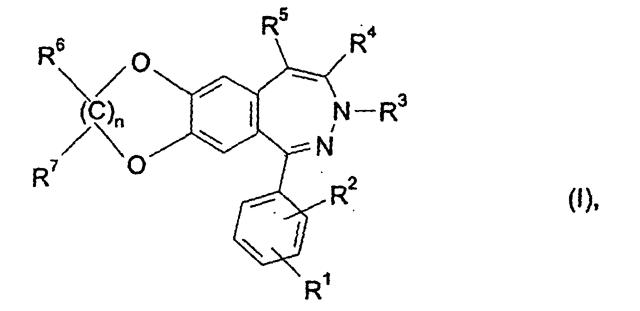
R¹ et R² sont identiques ou différents et représentent un hydrogène, un alkyle en C₁-C₆, un nitro, un halogène, le groupe -NR⁸-R⁹, ou un O-C₁₋₄-alkyle, ou -CF₃,
R³ représente le groupe , 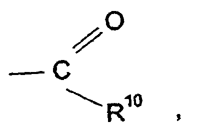
R⁴ représente un alkyle en C₁₋C₆ éventuellement substitué par un alcoxy en C₁₋₆, un halogène ou un alcanoyle en C₁₋₆,
R⁵ représente un hydrogène ou un alkyle en C₁₋C₆ éventuellement substitué par un alcoxy en C₁₋₆, un halogène ou un alcanoyle en C₁₋₆,
R⁶ et R⁷ sont identiques ou différents et représentent un hydrogène, un alkyle en C₁-C₆ éventuellement substitué par un alcoxy en C₁₋₆, un halogène ou un alcanoyle en C₁₋₆, ou un aryle en C₆₋₁₀ éventuellement substitué par un alcoxy en C₁₋₆, un halogène ou un alcanoyle en C₁₋₆,
R⁸ et R⁹ sont identiques ou différents et représentent un hydrogène, un alkyle en C₁₋C₆ ou le groupe 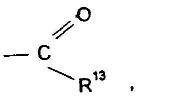
R¹⁰ représente un hydrogène, un alkyle en C₁₋C₆, éventuellement substitué par un alcoxy en C₁₋₆, un halogène ou un alcanoyle en C₁₋₆, un aryle en C₆₋₁₀ éventuellement substitué par un alcoxy en C₁₋₆, un halogène ou un alcanoyle en C₁₋₆, le groupe -NR¹¹R¹², un -O-C₁₋₆-alkyle, un cycloalkyle en C₃₋₇, un alcényle en C₂₋₆ ou un O-C₃₋₇-cycloalkyle,
R¹¹ et R¹² sont identiques ou différents et représentent un hydrogène, un alkyle en C₁-C₆ éventuellement substitué par un alcoxy en C₁₋₆, un halogène ou un alcanoyle en C₁₋₆, ou un aryle en C₆₋₁₀ éventuellement substitué par un alcoxy en C₁₋₆, un halogène ou un alcanoyle en C₁₋₆,
R¹³ représente un alkyle en C₁₋C₆ et
n vaut 1, 2 ou 3,
ainsi que leurs sels et isomères physiologiquement acceptables.

2. Composés de formule I selon la revendication 1, dans laquelle
R¹ représente un nitro ou un amino,
R² représente un hydrogène, un alkyle en C₁-C₆, un nitro, un halogène, le groupe -NR⁸R⁹, un O-C₁₋₄-alkyle ou -CF₃,
R³ représente le groupe 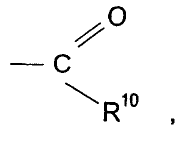
R⁴ représente un alkyle en C₁₋C₆ éventuellement substitué par un alcoxy en C₁₋₆, un halogène ou un alcanoyle en C₁₋₆,
R⁵ représente un hydrogène ou un alkyle en C₁₋C₆ éventuellement substitué par un alcoxy en C₁₋₆, un halogène ou un alcanoyle en C₁₋₆,
R⁶ et R⁷ sont identiques ou différents et représentent un hydrogène, un alkyle en C₁₋C₆ éventuellement substitué par un alcoxy en C₁₋₆, un halogène ou un alcanoyle en C₁₋₆, ou un aryle en C₆₋₁₀ éventuellement substitué par un alcoxy en C₁₋₆, un halogène ou un alcanoyle en C₁₋₆,
R⁸ et R⁹ sont identiques ou différents et représentent un hydrogène, un alkyle en C₁-C₆ ou le groupe 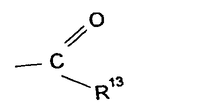
R¹⁰ représente un hydrogène, un alkyle en C₁₋C₆ éventuellement substitué par un alcoxy en C₁₋₆, un halogène ou un alcanoyle en C₁₋₆, un aryle en C₆₋₁₀ éventuellement substitué par un alcoxy en C₁₋₆, un halogène ou un alcanoyle en C₁₋₆, le groupe -NR¹¹R¹², un -O-C₁₋₆-alkyle, un cycloalkyle en C₃₋₇, un alcényle en C₂₋₆ ou un O-C₃₋₇-cycloalkyle,
R¹¹ et R¹² sont identiques ou différents et représentent un hydrogène, un alkyle en C₁-C₆ éventuellement substitué par un alcoxy en C₁₋₆, un halogène ou un alcanoyle en C₁₋₆, ou un aryle en C₆₋₁₀ éventuellement substitué par un alcoxy en C₁₋₆, un halogène ou un alcanoyle en C₁₋₆,
R¹³ représente un alkyle en C₁₋C₆ et
n vaut 1, 2 ou 3, ainsi que leurs sels et isomères physiologiquement acceptables.

3. 7-Acétyl-5-(4-aminophényl)-8-méthyl-7H-l,3-dioxolo[4,5-h][2,3]benzodiazépine,
7-méthylcarbamoyl-5-(4-aminophényl)-8-méthyl-7H-1,3-dioxolo[4,5-h][2,3]benzodiazépine, selon la revendication 1.

4. Composés de formule générale I selon la revendication 1, dans laquelle
R¹ représente un nitro ou un amino,
R² représente un hydrogène.
R³ représente le groupe 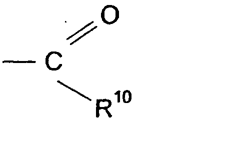
R⁴ représente un méthyle ou un éthyle,
R⁵, R⁶ et R⁷ représentent un hydrogène,
R¹⁰ représentent un hydrogène, un alkyle en C₁₋C₆, un phényle éventuellement substitué par un halogène, le groupe -NR¹¹R¹², un cycloalkyle en C₃₋₇, un alcényle en C₂₋₆, un -O-C₁₋₆-alkyle ou un alkyle en C₁₋₆ qui est mono- ou polysubstitué par un fluor,
R¹¹ et R¹² sont identiques ou différents et représentent un hydrogène, un alkyle en C₁-C₄ ou un phényle et
n vaut 1, 2 ou 3,
ainsi que leurs sels et isomères physiologiquement acceptables.

5. Procédé de préparation de composés de formule générale I selon la revendication 1, **caractérisé en ce qu'**un composé de formule générale II 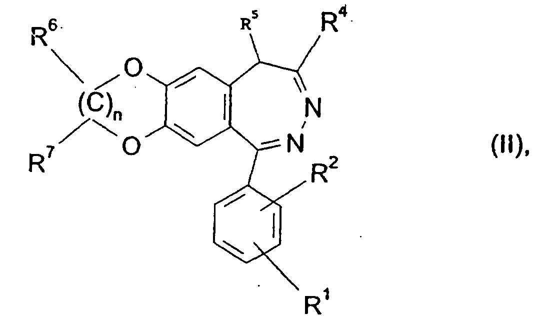 dans laquelle
R¹, R², R⁴, R⁵, R⁶, R⁷ et n ont les significations indiquées dans la formule générale I, est acylé, et le groupe nitro est éventuellement réduit catalytiquement sur R¹ et/ou R² et ensuite éventuellement acylé, alkylé, halogéné, le groupe carbamoyle est introduit avec des amines organiques ou le groupe ester est introduit avec des alcools, les isomères sont séparés ou les sels sont formés.

6. Médicament comprenant un composé de formule I selon l'une quelconque des revendications 1 à 4 et un ou plusieurs supports et/ou auxiliaires pharmaceutiquement habituels.

7. Utilisation d'un composé de formule générale I selon les revendications 1 à 4 pour la préparation d'un médicament destiné au traitement d'une maladie qui est influencée par l'hyperactivité d'acides aminés excitatoires.
